# EUROPEAN PATENT APPLICATION

(11) **EP 3 754 032 A1**
(43) Date of publication of application: **23.12.2020**
(21) Application number: 19754361.4
(22) Date of filing: 11.02.2019
(51) Int. Cl.: C12Q 1/689

(54) **METHOD FOR DIAGNOSIS OF STROKE THROUGH BACTERIAL METAGENOME ANALYSIS**

(30) Priority: 14.02.2018 KR 20180018793
(71) Applicant: MD Healthcare Inc., Seoul 03923 (KR)
(72) Inventor: KIM, Yoon-Keun, Paju-Si, Gyeonggi-do 10908 (KR)
(74) Representative: Dehns
(86) International application number: PCT/KR2019/001618
(87) International publication number: WO 2019/160284

(57) **Abstract**

The present invention relates to a method of diagnosing a stroke by bacterial metagenomic analysis, and more particularly, to a method of diagnosing a stroke by analyzing an increase or decrease in contents of specific bacteria-derived extracellular vesicles by performing bacterial metagenomic analysis using subject-derived samples. The extracellular vesicles secreted from bacteria present in the environment are absorbed into the body to have a direct influence on inflammation, and since a stroke is difficult to be diagnosed early before symptoms are shown, effective treatment is difficult. Therefore, by previously predicting the risk of the onset of a stroke through metagenomic analysis of bacterial extracellular vesicles using a human-derived sample according to the present invention, a stroke risk group may be diagnosed and predicted early to delay the time of onset or prevent the onset of a stroke with proper cure, and early diagnosis may be performed even after the onset of the disease, thereby reducing the incidence of a stroke and increasing a therapeutic effect.

<110>    MD Healthcare Inc.

<120>    Method for diagnosis of stroke using analysis of bacteria
         metagenome

<130>    MPCT18-087

<150>    KR 10-2018-0018793
<151>    2018-02-14

<160>    2

<170>    KoPatentIn 3.0

<210>    1
<211>    50
<212>    DNA
<213>    Artificial Sequence

<220>
<223>    16S_V3_F


<400>    1
tcgtcggcag cgtcagatgt gtataagaga cagcctacgg gnggcwgcag                50


<210>    2
<211>    55
<212>    DNA
<213>    Artificial Sequence

<220>
<223>    16S_V4_R


<400>    2
gtctcgtggg ctcggagatg tgtataagag acaggactac hvgggtatct aatcc          55

## Description

### [Technical Field]

The present invention relates to a method of diagnosing a stroke by bacterial metagenomic analysis, and more particularly, to a method of diagnosing a stroke by analyzing an increase or decrease in contents of specific bacteria-derived extracellular vesicles through bacterial metagenomic analysis using a subject-derived samples.

### [Background Art]

A stroke (apoplexy) is the generic term for symptoms of local neurological defect caused by abnormalities of abnormal blood flow to the brain. A stroke is the term for a symptom, and when referring to a medical condition, it is called cerebrovascular accident (CVA). In the United States, it is called cerebrovascular stroke. In terms of Chinese medicine, it is often referred to as palsy, which, however, is a broader expression. A stroke is broadly classified into cerebral infarction and cerebral hemorrhage.

The brain accounts for only 2% of the entire weight of the body, but blood flow to the brain is 15% of a cardiac output, and oxygen consumption is 20% of the entire oxygen consumption of the body. In addition, since the brain uses only glucose as an energy source, necrosis easily occurs even when energy supply is interrupted for a while. Therefore, abnormalities in cerebral blood flow are closely related to brain damage. The various risk factors for a stroke are known to be diverse, for example, constant risk factors including old age, being male, the family history of a stroke, and African and Asian races are known, and variable risk factors such as transient ischemic attacks, the history of a stroke, hypertension, coronary artery stenosis, diabetes, smoking, myocardial infarction, atrial fibrillation, the history of congestive heart failure, left ventricular failure, excessive alcohol consumption, and a blood clotting disorder are known. A stroke is a disease that has a high mortality rate, requires a lot of costs and time for treatment, and easily leaves impairments or disorders even if treated. In addition, in many cases, a stroke recurs after treatment. Therefore, most of all, prevention of a stroke is important.

Meanwhile, it is known that the number of microorganisms symbiotically living in the human body is 100 trillion which is 10 times the number of human cells, and the number of genes of microorganisms exceeds 100 times the number of human genes. A microbiota is a microbial community that includes bacteria, archaea, and eukaryotes present in a given habitat. The intestinal microbiota is known to play a vital role in human's physiological phenomena and significantly affect human health and diseases through interactions with human cells. Bacteria coexisting in human bodies secrete nanometer-sized vesicles to exchange information about genes, proteins, low molecular weight compound, and the like with other cells. The mucous membranes form a physical barrier membrane that does not allow particles with the size of 200 nm or more to pass therethrough, and thus bacteria symbiotically living in the mucous membranes are unable to pass therethrough, but bacteria-derived extracellular vesicles have a size of approximately 100 nm or less and thus relatively freely pass through the mucous membranes and are absorbed into the human body.

Metagenomics, also called environmental genomics, may be analytics for metagenomic data obtained from samples collected from the environment. Recently, the bacterial composition of human microbiota has been listed using a method based on 16s ribosomal RNA (16s rRNA) base sequences, and 16s rDNA base sequences, which are genes of 16s ribosomal RNA, are analyzed using a next generation sequencing (NGS) platform (Nature. 2007 Oct 18; 449(7164): 804-810). However, as for the occurrence of stroke, there is no report about a method of identifying, from a human-derived material such as blood, a causative factor of stroke by analysis of metagenomes present in bacteria-derived vesicles and of diagnosing stroke.

### [Disclosure]

### [Technical Problem]

The present inventors extracted genes from bacteria-derived extracellular vesicles present in blood as subject-derived samples and performed a metagenomic analysis in this regard in order to diagnose the causal factors and risk of stroke in advance, and as a result, identified bacteria-derived extracellular vesicles which may act as a causal factor of stroke, thereby completing the present invention based on this.

Therefore, an object of the present invention is to provide a method of providing information for diagnosing stroke through the metagenomic analysis of bacteria-derived extracellular vesicles.

However, the technical goals of the present invention are not limited to the aforementioned goals, and other unmentioned technical goals will be clearly understood by those of ordinary skill in the art from the following description.

### [Technical Solution]

To achieve the above-described object of the present invention, there is provided a method of providing information for stroke diagnosis, comprising the following processes:
(a) extracting DNAs from extracellular vesicles isolated from a subject sample;
(b) performing PCR on the extracted DNA using a pair of primers comprising SEQ ID NO: 1 and SEQ ID NO: 2; and
(c) comparing an increase or decrease in contents of bacteria-derived extracellular vesicles of the subject-derived sample with that of a normal individual-derived sample through sequencing of a product of the PCR.

The present invention also provides a method of diagnosing stroke, comprising the following processes:
(a) extracting DNAs from extracellular vesicles isolated from a subject sample;
(b) performing PCR on the extracted DNA using a pair of primers comprising SEQ ID NO: 1 and SEQ ID NO: 2; and
(c) comparing an increase or decrease in contents of bacteria-derived extracellular vesicles of the subject-derived sample with that of a normal individual-derived sample through sequencing of a product of the PCR.

The present invention also provides a method of predicting a risk for stroke, comprising the following processes:
(a) extracting DNAs from extracellular vesicles isolated from a subject sample;
(b) performing PCR on the extracted DNA using a pair of primers comprising SEQ ID NO: 1 and SEQ ID NO: 2; and
(c) comparing an increase or decrease in contents of bacteria-derived extracellular vesicles of the subject-derived sample with that of a normal individual-derived sample through sequencing of a product of the PCR.

In one embodiment of the present invention, in process (c), an increase or decrease in content of extracellular vesicles derived from one or more bacteria selected from the group consisting of the phylum *Deferribacteres*, the phylum *Verrucomicrobia*, the phylum *Tenericutes*, the phylum *Cyanobacteria*, the phylum *Bacteroidetes*, the phylum *Euryarchaeota*, the phylum TM7, the phylum *Planctomycetes*, the phylum GN02, and the phylum OD1 may be compared.

In another embodiment of the present invention, in process (c), an increase or decrease in content of extracellular vesicles derived from one or more bacteria selected from the group consisting of the class *Halobacteria*, the class *Deferribacteres*, the class *Verrucomicrobiae*, the class *Chloroplast*, the class *Mollicutes*, the class *Erysipelotrichi*, the class *Methanobacteria*, the class *Flavobacteriia*, the class *Planctomycetia*, the class TM7-1, and the class ZB2 may be compared.

In another embodiment of the present invention, in process (c), an increase or decrease in content of extracellular vesicles derived from one or more bacteria selected from the group consisting of the order *Halobacteriales,* the order *Deferribacterales*, the order *Streptophyta*, the order *Rhodocyclales*, the order *Verrucomicrobiales*, the order *Rickettsiales*, the order *Pasteurellales*, the order *Erysipelotrichales*, the order *Methanobacteriales*, the order *Legionellales*, and the order *Flavobacteriales* may be compared.

In another embodiment of the present invention, in process (c), an increase or decrease in content of extracellular vesicles derived from one or more bacteria selected from the group consisting of the family *Dermacoccaceae*, the family *Deferribacteraceae*, the family *Fusobacteriaceae*, the family *Lactobacillaceae*, the family *Rhodocyclaceae*, the family *Verrucomicrobiaceae*, the family *Oxalobacteraceae*, the family *Nocardioidaceae*, the family *Propionibacteriaceae*, the family *Pasteurellaceae*, the family *Erysipelotrichaceae*, the family *Corynebacteriaceae*, the family *Prevotellaceae*, the family *Comamonadaceae,* the family *Rikenellaceae,* the family *Odoribacteraceae*, the family *Micrococcaceae*, the family *Microbacteriaceae*, the family *Alcaligenaceae*, the family *Methanobacteriaceae*, the family *Flavobacteriaceae*, and the family *Cryomorphaceae* may be compared.

In another embodiment of the present invention, in process (c), an increase or decrease in content of extracellular vesicles derived from one or more bacteria selected from the group consisting of the genus *Ralstonia,* the genus *Geobacillus*, the genus *Psychrobacter,* the genus *Cupriavidus*, the genus *Chromohalobacter*, the genus *Dermacoccus,* the genus *Enterobacter*, the genus *Jeotgalicoccus*, the genus *Mucispirillum*, the genus rc4-4, the genus *Fusobacterium*, the genus *Citrobacter*, the genus *Kocuria*, the genus *Lactobacillus*, the genus *Akkermansia*, the genus *Adlercreutzia*, the genus *Veillonella*, the genus *Propionibacterium*, the genus *Haemophilus*, the genus *Catenibacterium*, the genus *Klebsiella*, the genus *Corynebacterium,* the genus *Acinetobacter*, the genus *Prevotella,* the genus *Collinsella*, the genus *Micrococcus*, the genus *Methanobrevibacter*, the genus *Sutterella*, the genus *Lachnobacterium*, the genus *Limnohabitans*, the genus *Polynucleobacter*, the genus *Rhodobacter*, the genus *Flavobacterium*, the genus *Fluviicola*, and the genus *Candidatus Aquiluna* may be compared.

In another embodiment of the present invention, in process (c), in comparison with the normal individual-derived sample, it is possible to diagnose an increase in the content of the following as stroke:
extracellular vesicles derived from one or more bacteria selected from the group consisting of the phylum *Bacteroidetes*, the phylum *Euryarchaeota*, the phylum TM7, the phylum *Planctomycetes*, the phylum GN02, and the phylum OD1,
extracellular vesicles derived from one or more bacteria selected from the group consisting of the class *Methanobacteria*, the class *Flavobacteriia*, the class *Planctomycetia*, the class TM7-1, and the class ZB2,
extracellular vesicles derived from one or more bacteria selected from the group consisting of the order *Methanobacteriales*, the order *Legionellales*, and the order *Flavobacteriales*,
extracellular vesicles derived from one or more bacteria selected from the group consisting of the family *Prevotellaceae*, the family *Comamonadaceae,* the family *Rikenellaceae*, the family *Odoribacteraceae,* the family *Micrococcaceae,* the family *Microbacteriaceae*, the family *Alcaligenaceae*, the family *Methanobacteriaceae*, the family *Flavobacteriaceae*, and the family *Cryomorphaceae*, or
extracellular vesicles derived from one or more bacteria selected from the group consisting of the genus *Prevotella*, the genus *Collinsella*, the genus *Micrococcus*, the genus *Methanobrevibacter*, the genus *Sutterella*, the genus *Lachnobacterium*, the genus *Limnohabitans*, the genus *Polynucleobacter*, the genus *Rhodobacter,* the genus *Flavobacterium*, the genus *Fluviicola*, and the genus *Candidatus Aquiluna.*

In another embodiment of the present invention, in process (c), in comparison with the normal individual-derived sample, it is possible to diagnose a decrease in the content of the following as stroke:
extracellular vesicles derived from one or more bacteria selected from the group consisting of the phylum *Deferribacteres*, the phylum *Verrucomicrobia*, the phylum *Tenericutes*, and the phylum *Cyanobacteria*,
extracellular vesicles derived from one or more bacteria selected from the group consisting of the class *Halobacteria,* the class *Deferribacteres*, the class *Verrucomicrobiae*, the class *Chloroplast*, the class *Mollicutes*, and the class *Erysipelotrichi*,
extracellular vesicles derived from one or more bacteria selected from the group consisting of the order *Halobacteriales*, the order *Deferribacterales*, the order *Streptophyta*, the order *Rhodocyclales*, the order *Verrucomicrobiales*, the order *Rickettsiales*, the order *Pasteurellales*, and the order *Erysipelotrichales*,
extracellular vesicles derived from one or more bacteria selected from the group consisting of the family *Dermacoccaceae*, the family *Deferribacteraceae*, the family *Fusobacteriaceae*, the family *Lactobacillaceae*, the family *Rhodocyclaceae*, the family *Verrucomicrobiaceae*, the family *Oxalobacteraceae,* the family *Nocardioidaceae*, the family *Propionibacteriaceae*, the family *Pasteurellaceae*, the family *Erysipelotrichaceae*, and the family *Corynebacteriaceae*, or
extracellular vesicles derived from one or more bacteria selected from the group consisting of the genus *Ralstonia*, the genus *Geobacillus*, the genus *Psychrobacter*, the genus *Cupriavidus*, the genus *Chromohalobacter*, the genus *Dermacoccus*, the genus *Enterobacter*, the genus *Jeotgalicoccus,* the genus *Mucispirillum*, the genus rc4-4, the genus *Fusobacterium*, the genus *Citrobacter*, the genus *Kocuria,* the genus *Lactobacillus*, the genus *Akkermansia*, the genus *Adlercreutzia*, the genus *Veillonella*, the genus *Propionibacterium*, the genus *Haemophilus*, the genus *Catenibacterium*, the genus *Klebsiella*, the genus *Corynebacterium*, and the genus *Acinetobacter.*

In another embodiment of the present invention, in process (c), in comparison with the normal individual-derived sample, stroke may be diagnosed when the content of vesicles derived from bacteria of the genus *Collinsella* and the genus *Flavobacterium* is increased, and the content of vesicles derived from bacteria of the genus *Lactobacillus* and the genus *Propionibacterium* is decreased.

In another embodiment of the present invention, in process (c), in comparison with the normal individual-derived sample, stroke may be diagnosed when the content of vesicles derived from bacteria of the genus *Collinsella* and the genus *Flavobacterium* is increased, and the content of vesicles derived from bacteria of the genus *Lactobacillus* and the genus *Propionibacterium* is decreased; and
the content of extracellular vesicles derived from one or more bacteria selected from the group consisting of the phylum *Bacteroidetes,* the phylum *Euryarchaeota*, and the phylum *Planctomycetes*,
extracellular vesicles derived from one or more bacteria selected from the group consisting of the class *Methanobacteria*, the class *Flavobacteriia*, and the class *Planctomycetia*,
extracellular vesicles derived from one or more bacteria selected from the group consisting of the order *Methanobacteriales*, the order *Legionellales*, and the order *Flavobacteriales,*
extracellular vesicles derived from one or more bacteria selected from the group consisting of the family *Prevotellaceae*, the family *Comamonadaceae,* the family *Rikenellaceae*, the family *Odoribacteraceae*, the family *Micrococcaceae*, the family *Microbacteriaceae*, the family *Alcaligenaceae*, the family *Methanobacteriaceae*, the family *Flavobacteriaceae*, and the family *Cryomorphaceae*, or
extracellular vesicles derived from one or more bacteria selected from the group consisting of the genus *Prevotella*, the genus *Micrococcus,* the genus *Methanobrevibacter*, the genus *Sutterella*, the genus *Lachnobacterium*, the genus *Limnohabitans*, the genus *Polynucleobacter*, the genus *Rhodobacter*, the genus *Fluviicola*, and the genus *Candidatus Aquiluna* is increased.

In another embodiment of the present invention, in process (c), in comparison with the normal individual-derived sample, stroke may be diagnosed when the content of vesicles derived from bacteria of the genus *Collinsella* and the genus *Flavobacterium* is increased, and the content of vesicles derived from bacteria of the genus *Lactobacillus* and the genus *Propionibacterium* is decreased; and
the content of extracellular vesicles derived from one or more bacteria selected from the group consisting of the phylum *Deferribacteres*, the phylum *Verrucomicrobia*, the phylum *Tenericutes*, and the phylum *Cyanobacteria*,
extracellular vesicles derived from one or more bacteria selected from the group consisting of the class *Halobacteria,* the class *Deferribacteres*, the class *Verrucomicrobiae*, the class *Chloroplast*, the class *Mollicutes,* and the class *Erysipelotrichi,*
extracellular vesicles derived from one or more bacteria selected from the group consisting of the order *Halobacteriales,* the order *Deferribacterales*, the order *Streptophyta*, the order *Rhodocyclales,* the order *Verrucomicrobiales*, the order *Rickettsiales*, the order *Pasteurellales*, and the order *Erysipelotrichales*,
extracellular vesicles derived from one or more bacteria selected from the group consisting of the family *Dermacoccaceae*, the family *Deferribacteraceae,* the family *Fusobacteriaceae*, the family *Lactobacillaceae*, the family *Rhodocyclaceae*, the family *Verrucomicrobiaceae*, the family *Oxalobacteraceae*, the family *Nocardioidaceae*, the family *Propionibacteriaceae*, the family *Pasteurellaceae*, the family *Erysipelotrichaceae*, and the family *Corynebacteriaceae*, or
extracellular vesicles derived from one or more bacteria selected from the group consisting of the genus *Ralstonia*, the genus *Geobacillus*, the genus *Psychrobacter*, the genus *Cupriavidus*, the genus *Chromohalobacter,* the genus *Dermacoccus*, the genus *Enterobacter,* the genus *Jeotgalicoccus*, the genus *Mucispirillum*, the genus *Fusobacterium,* the genus *Citrobacter*, the genus *Kocuria*, the genus *Akkermansia*, the genus *Adlercreutzia*, the genus *Veillonella*, the genus *Haemophilus*, the genus *Catenibacterium,* the genus *Klebsiella*, the genus *Corynebacterium*, and the genus *Acinetobacter* is decreased.

In another embodiment of the present invention, the subject sample may be blood.

In another embodiment of the present invention, the blood may be whole blood, serum, plasma, or blood mononuclear cells.

### [Advantageous Effects]

Extracellular vesicles secreted from bacteria present in the environment can be absorbed into the body and have a direct influence on the onset of an inflammation, and since it is difficult to enable early diagnosis of a stroke before symptoms are shown, it is difficult to effectively treat a stroke. Therefore, stroke risk groups can be diagnosed and predicted early by previously diagnosing the causative factor and the risk of the onset of a stroke through the metagenomic analysis of bacteria-derived extracellular vesicles using a human-derived sample according to the present invention, the time of onset can be delayed or the onset of the disease can be prevented with proper care, and since a stroke can be diagnosed early after onset, the incidence of a stroke can be reduced and a therapeutic effect can increase. In addition, the progress of the disease can be improved or the recurrence of the disease can be prevented by identifying causative factors through a metagenomic analysis on patients diagnosed with a stroke to avoid exposure to the relevant factors.

### [Description of Drawings]

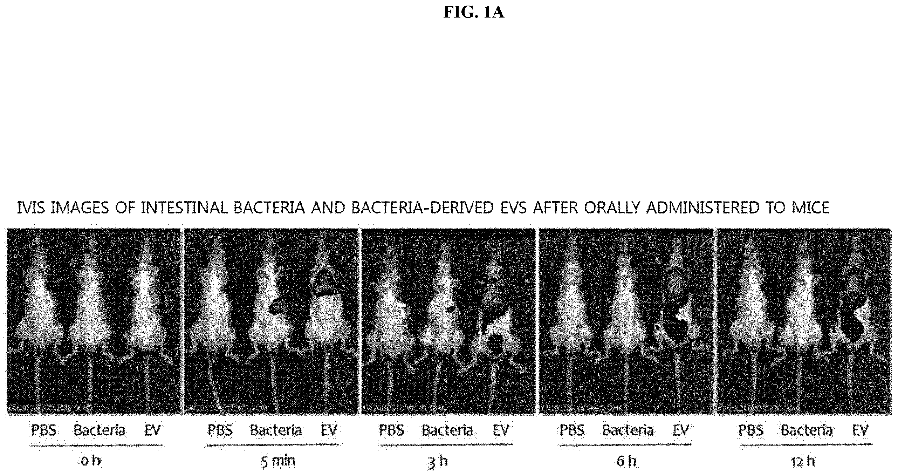

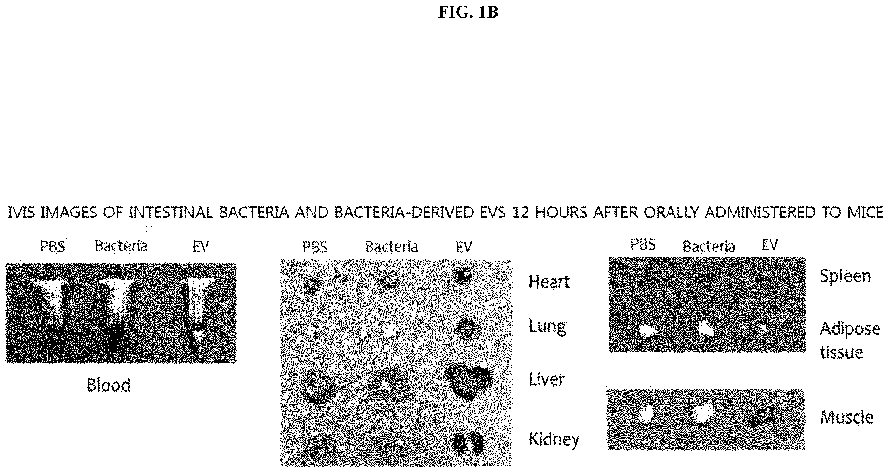

FIG. 1A illustrates images showing the distribution pattern of bacteria and extracellular vesicles over time after intestinal bacteria and bacteria-derived extracellular vesicles (EVs) were orally administered to mice, and FIG. 1B illustrates images showing the distribution pattern of bacteria and EVs after being orally administered to mice and, at 12 hours, blood and various organs were extracted.

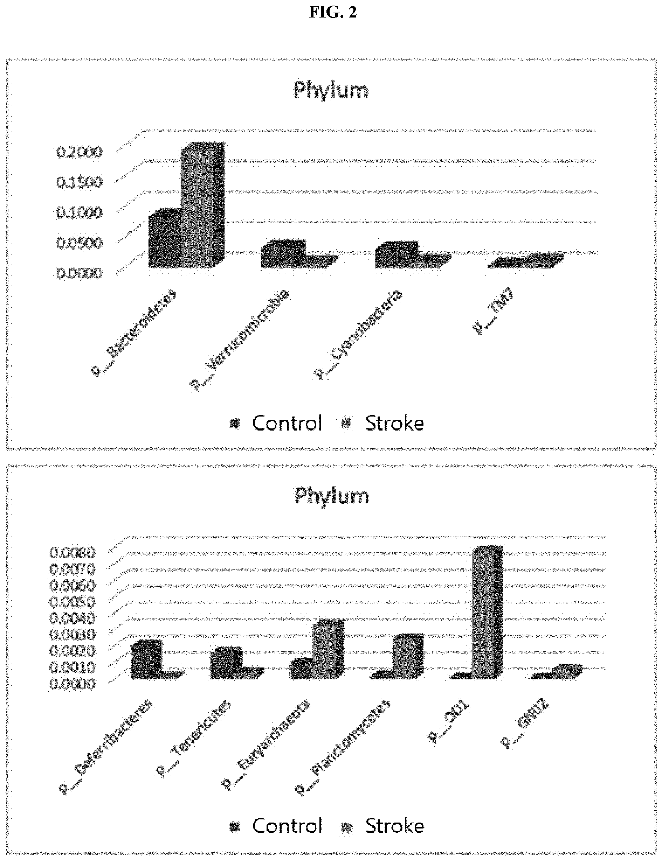

FIG. 2 is a result showing the distribution of bacteria-derived extracellular vesicles (EVs), which is significant in diagnostic performance at the phylum level by isolating bacteria-derived vesicles from blood of a patient with stroke and a normal individual, and then performing a metagenomic analysis.

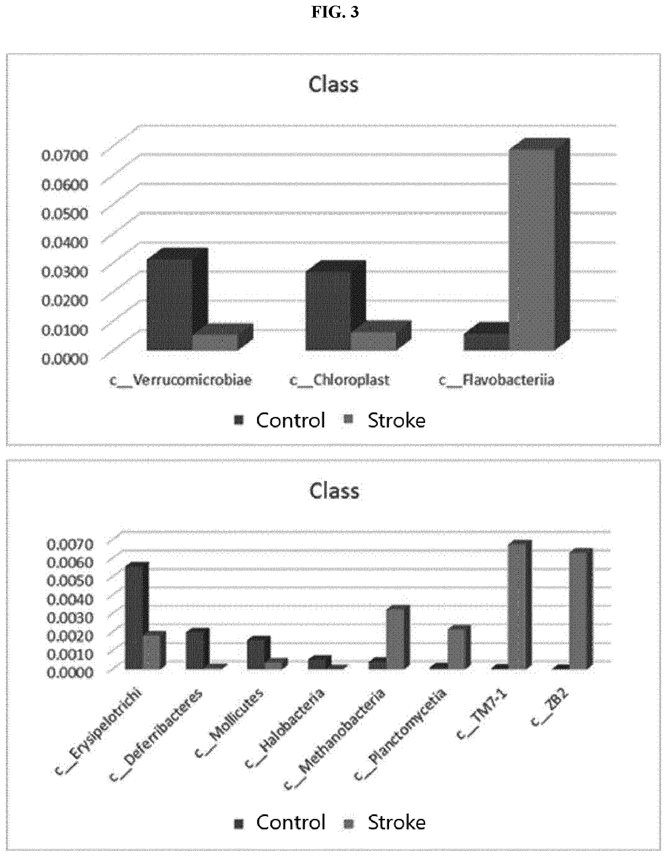

FIG. 3 is a result showing the distribution of bacteria-derived extracellular vesicles (EVs), which is significant in diagnostic performance at the class level by isolating bacteria-derived vesicles from blood of a patient with stroke and a normal individual, and then performing a metagenomic analysis.

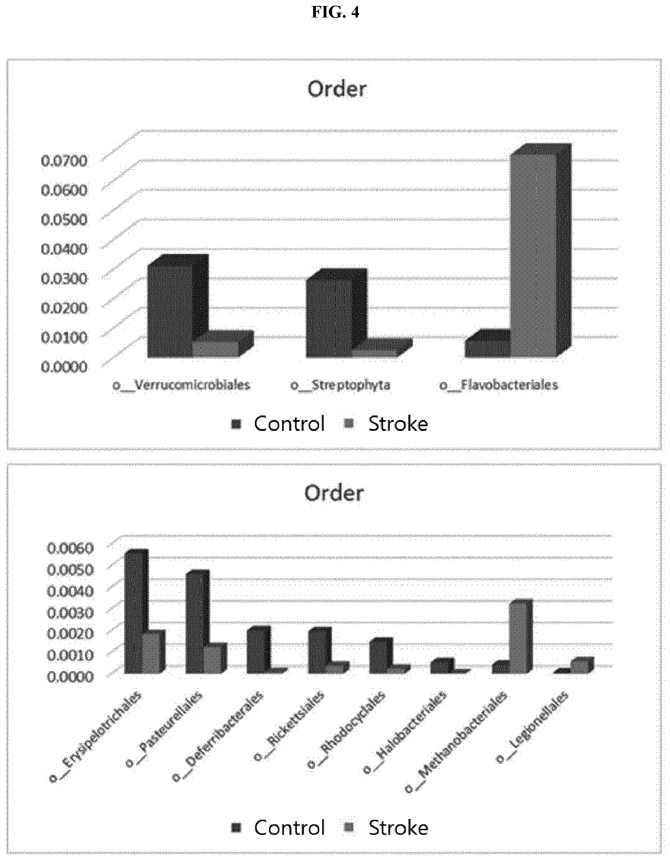

FIG. 4 is a result showing the distribution of bacteria-derived extracellular vesicles (EVs), which is significant in diagnostic performance at the order level by isolating bacteria-derived vesicles from blood of a patient with stroke and a normal individual, and then performing a metagenomic analysis.

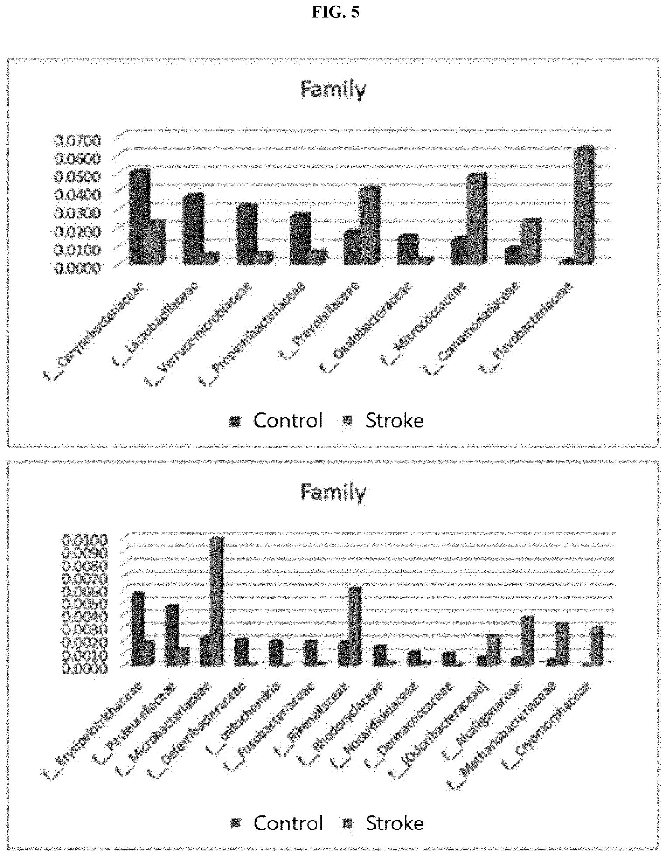

FIG. 5 is a result showing the distribution of bacteria-derived extracellular vesicles (EVs), which is significant in diagnostic performance at the family level by isolating bacteria-derived vesicles from blood of a patient with stroke and a normal individual, and then performing a metagenomic analysis.

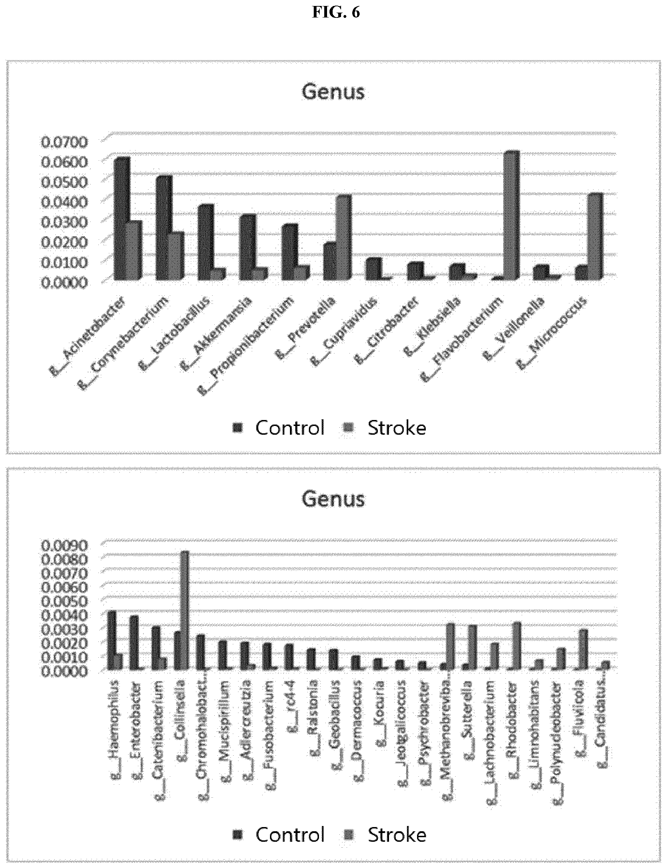

FIG. 6 is a result showing the distribution of bacteria-derived extracellular vesicles (EVs), which is significant in diagnostic performance at the genus level by isolating bacteria-derived vesicles from blood of a patient with stroke and a normal individual, and then performing a metagenomic analysis.

### [Best Mode]

The present invention relates to a method of diagnosing stroke through bacterial metagenomic analysis. The inventors of the present invention extracted genes from bacteria-derived extracellular vesicles using a subject-derived sample, performed metagenomic analysis thereon, and identified bacteria-derived extracellular vesicles capable of acting as a causative factor of stroke.

Therefore, the present invention provides a method of providing information for diagnosing stroke, the method comprising:
(a) extracting DNAs from extracellular vesicles isolated from a subject sample;
(b) performing PCR on the extracted DNA using a pair of primers comprising SEQ ID NO: 1 and SEQ ID NO: 2; and
(c) comparing an increase or decrease in contents of bacteria-derived extracellular vesicles of the subject-derived sample with that of a normal individual-derived sample through sequencing of a product of the PCR.

The term "stroke diagnosis" as used herein refers to determining whether a patient has a risk for stroke, whether the risk for stroke is relatively high, or whether stroke has already occurred. The method of the present invention may be used to delay the onset of stroke through special and appropriate care for a specific patient, which is a patient having a high risk for stroke or prevent the onset of stroke. In addition, the method may be clinically used to determine treatment by selecting the most appropriate treatment method through early diagnosis of stroke.

The term "metagenome" as used herein refers to the total of genomes including all viruses, bacteria, fungi, and the like in isolated regions such as soil, the intestines of animals, and the like, and is mainly used as a concept of genomes that explains identification of many microorganisms at once using a sequencer to analyze non-cultured microorganisms. In particular, a metagenome does not refer to a genome of one species, but refers to a mixture of genomes, including genomes of all species of an environmental unit. This term originates from the view that, when defining one species in a process in which biology is advanced into omics, various species as well as existing one species functionally interact with each other to form a complete species. Technically, it is the subject of techniques that analyzes all DNAs and RNAs regardless of species using rapid sequencing to identify all species in one environment and verify interactions and metabolism. In the present invention, bacterial metagenomic analysis is performed using bacteria-derived extracellular vesicles isolated from, for example, serum.

The term "bacteria-derived vesicles" used herein include extracellular vesicles secreted from bacteria and Archaea, but the present invention is not limited thereto.

In the present invention, the subject sample may be blood, and the blood may be whole blood, serum, plasma, or blood mononuclear cells, but the present invention is not limited thereto.

In an embodiment of the present invention, metagenomic analysis is performed on the bacteria-derived extracellular vesicles, and bacteria-derived vesicles capable of acting as a cause of the onset of stroke were actually identified by analysis at phylum, class, order, family, and genus levels.

More particularly, in one embodiment of the present invention, as a result of performing bacterial metagenomic analysis on vesicles present in subject-derived blood samples at a phylum level, the content of extracellular vesicles derived from bacteria belonging to the phylum *Deferribacteres*, the phylum *Verrucomicrobia*, the phylum *Tenericutes*, the phylum *Cyanobacteria*, the phylum *Bacteroidetes*, the phylum *Euryarchaeota*, the phylum TM7, the phylum *Planctomycetes*, the phylum GN02, and the phylum OD1 was significantly different between stroke patients and normal individuals (see Example 4).

More particularly, in one embodiment of the present invention, as a result of performing bacterial metagenomic analysis on vesicles present in subject-derived blood samples at a class level, the content of extracellular vesicles derived from bacteria belonging to the class *Halobacteria*, the class *Deferribacteres*, the class *Verrucomicrobiae*, the class *Chloroplast,* the class *Mollicutes*, the class *Erysipelotrichi*, the class *Methanobacteria*, the class *Flavobacteriia*, the class *Planctomycetia*, the class TM7-1, and the class ZB2 was significantly different between stroke patients and normal individuals (see Example 4).

More particularly, in one embodiment of the present invention, as a result of performing bacterial metagenomic analysis on vesicles present in subj ect-derived blood samples at an order level, the content of extracellular vesicles derived from bacteria belonging to the order *Halobacteriales*, the order *Deferribacterales*, the order *Streptophyta*, the order *Rhodocyclales*, the order *Verrucomicrobiales*, the order *Rickettsiales*, the order *Pasteurellales,* the order *Erysipelotrichales*, the order *Methanobacteriales*, the order *Legionellales*, and the order *Flavobacteriales* was significantly different between stroke patients and normal individuals (see Example 4).

More particularly, in one embodiment of the present invention, as a result of performing bacterial metagenomic analysis on vesicles present in subj ect-derived blood samples at a family level, the content of extracellular vesicles derived from bacteria belonging to the family *Dermacoccaceae*, the family *Deferribacteraceae*, the family *Fusobacteriaceae*, the family *Lactobacillaceae*, the family *Rhodocyclaceae*, the family *Verrucomicrobiaceae*, the family *Oxalobacteraceae*, the family *Nocardioidaceae*, the family *Propionibacteriaceae*, the family *Pasteurellaceae*, the family *Erysipelotrichaceae*, the family *Corynebacteriaceae*, the family *Prevotellaceae*, the family *Comamonadaceae*, the family *Rikenellaceae*, the family *Odoribacteraceae*, the family *Micrococcaceae*, the family *Microbacteriaceae*, the family *Alcaligenaceae*, the family *Methanobacteriaceae*, the family *Flavobacteriaceae*, and the family *Cryomorphaceae* was significantly different between stroke patients and normal individuals (see Example 4).

More particularly, in one embodiment of the present invention, as a result of performing bacterial metagenomic analysis on vesicles present in subject-derived blood samples at a genus level, the content of extracellular vesicles derived from bacteria belonging to the genus *Ralstonia*, the genus *Geobacillus*, the genus *Psychrobacter*, the genus *Cupriavidus*, the genus *Chromohalobacter*, the genus *Dermacoccus*, the genus *Enterobacter*, the genus *Jeotgalicoccus*, the genus *Mucispirillum*, the genus rc4-4, the genus *Fusobacterium*, the genus *Citrobacter*, the genus *Kocuria*, the genus *Lactobacillus*, the genus *Akkermansia*, the genus *Adlercreutzia*, the genus *Veillonella*, the genus *Propionibacterium*, the genus *Haemophilus*, the genus *Catenibacterium*, the genus *Klebsiella*, the genus *Corynebacterium*, the genus *Acinetobacter*, the genus *Prevotella*, the genus *Collinsella,* the genus *Micrococcus*, the genus *Methanobrevibacter*, the genus *Sutterella*, the genus *Lachnobacterium*, the genus *Limnohabitans*, the genus *Polynucleobacter*, the genus *Rhodobacter*, the genus *Flavobacterium*, the genus *Fluviicola*, and the genus *Candidatus Aquiluna* was significantly different between stroke patients and normal individuals (see Example 4).

Through the results of the examples, it was confirmed that distribution variables of the identified bacteria-derived extracellular vesicles could be usefully used for the prediction of the onset of stroke.

### [Modes of the Invention]

Hereinafter, the present invention will be described with reference to exemplary examples to aid in understanding of the present invention. However, these examples are provided only for illustrative purposes and are not intended to limit the scope of the present invention.

### [Examples]

### Example 1. Analysis of In Vivo Absorption, Distribution, and Excretion Patterns of Intestinal Bacteria and Bacteria-Derived Vesicles

To evaluate whether intestinal bacteria and bacteria-derived vesicles are absorbed systemically through the mucosa, an experiment was performed using the following method. 50 µg of each of intestinal bacteria and the intestinal bacteria-derived extracellular vesicles (EVs), labeled with fluorescence, were administered to the gastrointestinal tracts of mice, and fluorescence was measured at 0 h, and after 5 min, 3 h, 6 h, and 12 h. As a result of observing the entire images of mice, as illustrated in FIG. 1A, the bacteria were not systematically absorbed when administered, while the bacteria-derived EVs were systematically absorbed at 5 min after administration, and, at 3 h after administration, fluorescence was strongly observed in the bladder, from which it was confirmed that the EVs were excreted via the urinary system, and were present in the bodies up to 12 h after administration.

After intestinal bacteria and intestinal bacteria-derived extracellular vesicles were systematically absorbed, to evaluate a pattern of invasion of intestinal bacteria and the bacteria-derived EVs into various organs in the human body after being systematically absorbed, 50 µg of each of the bacteria and bacteria-derived EVs, labeled with fluorescence, were administered using the same method as that used above, and then, at 12 h after administration, blood, heart, lungs, liver, kidneys, spleen, adipose tissue, and muscle were extracted from each mouse. As a result of observing fluorescence in the extracted tissues, as illustrated in FIG. 1B, it was confirmed that the intestinal bacteria were not absorbed into each organ, while the intestinal bacteria-derived EVs were distributed in the blood, heart, lungs, liver, kidneys, spleen, adipose tissue, and muscle.

### Example 2. Vesicle Isolation and DNA Extraction from Blood

To isolate vesicles and extract DNA, from blood, first, blood was added to a 10 ml tube and centrifuged at 3,500 x g and 4 °C for 10 min to precipitate a suspension, and only a supernatant was collected, which was then placed in a new 10 ml tube. The collected supernatant was filtered using a 0.22 µm filter to remove bacteria and impurities, and then placed in centrifugal filters (50 kD) and centrifuged at 1500 x g and 4 °C for 15 min to discard materials with a smaller size than 50 kD, and then concentrated to 10 ml. Once again, bacteria and impurities were removed therefrom using a 0.22 µm filter, and then the resulting concentrate was subjected to ultra-high speed centrifugation at 150,000 x g and 4 °C for 3 hours by using a Type 90ti rotor to remove a supernatant, and the agglomerated pellet was dissolved with phosphate-buffered saline (PBS), thereby obtaining vesicles.

100 µl of the extracellular vesicles isolated from the blood according to the above-described method was boiled at 100 °C to allow the internal DNA to come out of the lipid and then cooled on ice for 5 minutes. Next, the resulting vesicles were centrifuged at 10,000 x g and 4 °C for 30 minutes to remove the remaining suspension, only the supernatant was collected, and then the amount of DNA extracted was quantified using a NanoDrop sprectrophotometer. In addition, to verify whether bacteria-derived DNA was present in the extracted DNA, PCR was performed using 16s rDNA primers shown in Table 1 below.

**[Table 1]**

| Primer | | Sequence | SEQ ID NO. |
|---|---|---|---|
| 16S rDNA | 16S_V3_F | 5'-TCGTCGGCAGCGTC AGATGTGTATAAGAG ACAGCCTACGGGNGG CWGCAG-3' | 1 |
| | 16S_V4_R | 5'-GTCTCGTGGGCTCG GAGATGTGTATAAGA GACAGGACTACHVGG GTATCTAATCC-3' | 2 |

### Example 3. Metagenomic Analysis Using DNA Extracted from Blood

DNA was extracted using the same method as that used in Example 2, and then PCR was performed thereon using 16S rDNA primers shown in Table 1 to amplify DNA, followed by sequencing (Illumina MiSeq sequencer). The results were output as standard flowgram format (SFF) files, and the SFF files were converted into sequence files (.fasta) and nucleotide quality score files using GS FLX software (v2.9), and then credit rating for reads was identified, and portions with a window (20 bps) average base call accuracy of less than 99% (Phred score <20) were removed. After removing the low-quality portions, only reads having a length of 300 bps or more were used (Sickle version 1.33), and for operational taxonomy unit (OTU) analysis, clustering was performed using UCLUST and USEARCH according to sequence similarity. In particular, clustering was performed based on sequence similarity values of 94% for genus, 90% for family, 85% for order, 80% for class, and 75% for phylum, and phylum, class, order, family, and genus levels of each OTU were classified, and bacteria with a sequence similarity of 97% or more were analyzed (QIIME) using 16S DNA sequence databases (108,453 sequences) of BLASTN and GreenGenes.

### Example 4. Stroke Diagnostic Model Based on Metagenomic Analysis of Bacteria-Derived EVs Isolated from Blood

EVs were isolated from blood samples of 115 stroke patients and 109 normal individuals, the two groups matched in age and gender, and then metagenomic sequencing was performed thereon using the method of Example 3. For the development of a diagnostic model, first, a strain exhibiting a p value of less than 0.05 between two groups in a t-test and a difference of two-fold or more between two groups was selected, and then an area under curve (AUC), sensitivity, and specificity, which are diagnostic performance indexes, were calculated by logistic regression analysis.

As a result of analyzing bacteria-derived EVs in blood at a phylum level, a diagnostic model developed using bacteria belonging to the phylum *Deferribacteres,* the phylum *Verrucomicrobia*, the phylum *Tenericutes*, the phylum *Cyanobacteria*, the phylum *Bacteroidetes*, the phylum *Euryarchaeota*, the phylum TM7, the phylum *Planctomycetes*, the phylum GN02, and the phylum OD1 as a biomarker exhibited significant diagnostic performance for stroke (see Table 2 and FIG. 2).

**[Table 2]**

| | Control | | Stroke | | t-test | | Training Set | | | Test Set | | |
|---|---|---|---|---|---|---|---|---|---|---|---|---|
| Taxon | Mean | SD | Mean | SD | p-value | Ratio | AUC | sensitivity | specificity | AUC | sensitivity | specificity |
| p_Deferribacteres | 0.0020 | 0.0041 | 0.0001 | 0.0007 | 0.0000 | 0.03 | 0.66 | 0.31 | 0.89 | 0.61 | 0.53 | 0.89 |
| p_Verrucomicrobia | 0.0314 | 0.0441 | 0.0057 | 0.0185 | 0.0000 | 0.18 | 0.85 | 0.70 | 0.86 | 0.77 | 0.59 | 0.81 |
| p_Tenericutes | 0.0016 | 0.0036 | 0.0004 | 0.0015 | 0.0015 | 0.23 | 0.67 | 0.53 | 0.77 | 0.48 | 0.34 | 0.67 |
| p_Cyanobacteria | 0.0281 | 0.0622 | 0.0073 | 0.0298 | 0.0020 | 0.26 | 0.64 | 0.40 | 0.75 | 0.48 | 0.38 | 0.58 |
| p_Bacteroidetes | 0.0823 | 0.0642 | 0.1912 | 0.1502 | 0.0000 | 2.32 | 0.69 | 0.81 | 0.58 | 0.70 | 0.84 | 0.58 |
| p_Euryarchaeota | 0.0009 | 0.0026 | 0.0032 | 0.0077 | 0.0029 | 3.48 | 0.61 | 0.58 | 0.52 | 0.40 | 0.50 | 0.33 |
| p_TM7 | 0.0021 | 0.0048 | 0.0083 | 0.0167 | 0.0002 | 3.99 | 0.61 | 0.75 | 0.44 | 0.68 | 0.66 | 0.56 |
| p_Planetomycetes | 0.0001 | 0.0006 | 0.0024 | 0.0064 | 0.0003 | 20.57 | 0.68 | 0.75 | 0.47 | 0.50 | 0.59 | 0.42 |
| p_GN02 | 0.0000 | 0.0000 | 0.0005 | 0.0020 | 0.0099 | 111.46 | 0.64 | 0.62 | 0.52 | 0.38 | 0.53 | 0.25 |
| p_OD1 | 0.0000 | 0.0002 | 0.0077 | 0.0129 | 0.0000 | 196.93 | 0.83 | 0.96 | 0.61 | 0.75 | 1.00 | 0.58 |

As a result of analyzing bacteria-derived EVs in blood at a class level, a diagnostic model developed using bacteria belonging to the class *Halobacteria*, the class *Deferribacteres*, the class *Verrucomicrobiae*, the class *Chloroplast*, the class *Mollicutes*, the class *Erysipelotrichi*, the class *Methanobacteria*, the class *Flavobacteriia*, the class *Planctomycetia*, the class TM7-1, and the class ZB2 as a biomarker exhibited significant diagnostic performance for stroke (see Table 3 and FIG. 3).

**[Table 3]**

| | Control | | Stroke | | t-test | | Training Set | | | Test Set | | |
|---|---|---|---|---|---|---|---|---|---|---|---|---|
| Taxon | Mean | SD | Mean | SD | p-value | Ratio | AUC | sensitivity | specificity | AUC | sensitivity | specificity |
| c_ Halobacteria | 0.0005 | 0.0020 | 0.0000 | 0.0000 | 0.0069 | 0.00 | 0.59 | 0.15 | 1.00 | 0.47 | 0.03 | 1.00 |
| c_ Deferribacteres | 0.0020 | 0.0041 | 0.0001 | 0.0007 | 0.0000 | 0.03 | 0.71 | 0.32 | 0.99 | 0.61 | 0.21 | 1.00 |
| c_ Verrucomicrobiae | 0.0311 | 0.0439 | 0.0053 | 0.0184 | 0.0000 | 0.17 | 0.84 | 0.55 | 0.90 | 0.84 | 0.56 | 0.91 |
| c_Chloroplast | 0.0269 | 0.0622 | 0.0062 | 0.0296 | 0.0020 | 0.23 | 0.71 | 0.35 | 0.93 | 0.68 | 0.47 | 0.88 |
| c_ Mollicutes | 0.0016 | 0.0036 | 0.0004 | 0.0015 | 0.0017 | 0.24 | 0.65 | 0.29 | 0.88 | 0.69 | 0.38 | 0.91 |
| c_ Erysipelotrichi | 0.0056 | 0.0104 | 0.0018 | 0.0054 | 0.0010 | 0.33 | 0.61 | 0.29 | 0.86 | 0.69 | 0.41 | 0.94 |
| c_ Methanobacteria | 0.0004 | 0.0017 | 0.0032 | 0.0077 | 0.0002 | 7.88 | 0.60 | 0.83 | 0.31 | 0.55 | 0.88 | 0.26 |
| c_ Flavobacteriia | 0.0057 | 0.0082 | 0.0688 | 0.0915 | 0.0000 | 12.12 | 0.71 | 0.89 | 0.54 | 0.61 | 0.94 | 0.47 |
| c_ Planctomycetia | 0.0001 | 0.0006 | 0.0021 | 0.0063 | 0.0008 | 21.55 | 0.61 | 0.97 | 0.25 | 0.57 | 0.94 | 0.24 |
| c_TM7-1 | 0.0000 | 0.0004 | 0.0068 | 0.0166 | 0.0000 | 174.14 | 0.71 | 0.95 | 0.37 | 0.81 | 0.91 | 0.59 |
| c_ZB2 | 0.0000 | 0.0001 | 0.0063 | 0.0112 | 0.0000 | 530.99 | 0.81 | 0.97 | 0.53 | 0.75 | 1.00 | 0.50 |

As a result of analyzing bacteria-derived EVs in blood at an order level, a diagnostic model developed using bacteria belonging to the order *Halobacteriales*, the order *Deferribacterales*, the order *Streptophyta,* the order *Rhodocyclales*, the order *Verrucomicrobiales*, the order *Rickettsiales*, the order *Pasteurellales*, the order *Erysipelotrichales*, the order *Methanobacteriales*, the order *Legionellales*, and the order *Flavobacteriales* as a biomarker exhibited significant diagnostic performance for stroke (see Table 4 and FIG. 4).

**[Table 4]**

| | Control | | Stroke | | t-test | | Training Set | | | Test Set | | |
|---|---|---|---|---|---|---|---|---|---|---|---|---|
| Taxon | Mean | SD | Mean | SD | p-value | Ratio | AUC | sensitivity | specificity | AUC | sensitivity | specificity |
| o_ Halobacteriales | 0.0005 | 0.0020 | 0.0000 | 0.0000 | 0.0069 | 0.00 | 0.60 | 0.43 | 0.70 | 0.48 | 0.44 | 0.44 |
| o_ Deferribacterales | 0.0020 | 0.0041 | 0.0001 | 0.0007 | 0.0000 | 0.03 | 0.66 | 0.31 | 0.89 | 0.61 | 0.53 | 0.89 |
| o_Streptophyta | 0.0263 | 0.0622 | 0.0025 | 0.0132 | 0.0002 | 0.10 | 0.75 | 0.40 | 0.89 | 0.68 | 0.41 | 0.92 |
| o_ Rhodocyclales | 0.0015 | 0.0045 | 0.0002 | 0.0010 | 0.0056 | 0.15 | 0.61 | 0.48 | 0.67 | 0.43 | 0.38 | 0.47 |
| o_Verrucomicrobiales | 0.0311 | 0.0439 | 0.0053 | 0.0184 | 0.0000 | 0.17 | 0.87 | 0.71 | 0.89 | 0.80 | 0.59 | 0.78 |
| o_Rickettsiales | 0.0019 | 0.0058 | 0.0004 | 0.0015 | 0.0068 | 0.18 | 0.61 | 0.40 | 0.71 | 0.44 | 0.34 | 0.67 |
| o_Pasteurellales | 0.0046 | 0.0070 | 0.0012 | 0.0037 | 0.0000 | 0.27 | 0.72 | 0.44 | 0.84 | 0.62 | 0.50 | 0.81 |
| o_ Erysipelotrichales | 0.0056 | 0.0104 | 0.0018 | 0.0054 | 0.0010 | 0.33 | 0.72 | 0.53 | 0.80 | 0.46 | 0.31 | 0.67 |
| o_Methanobacteriales | 0.0004 | 0.0017 | 0.0032 | 0.0077 | 0.0002 | 7.88 | 0.64 | 0.66 | 0.52 | 0.43 | 0.56 | 0.33 |
| o_ Legionellales | 0.0001 | 0.0003 | 0.0006 | 0.0018 | 0.0033 | 10.46 | 0.63 | 0.66 | 0.47 | 0.45 | 0.56 | 0.36 |
| o_Flavobacteriales | 0.0057 | 0.0082 | 0.0688 | 0.0915 | 0.0000 | 12.12 | 0.75 | 0.88 | 0.51 | 0.66 | 0.84 | 0.56 |

As a result of analyzing bacteria-derived EVs in blood at a family level, a diagnostic model developed using bacteria belonging to the family *Dermacoccaceae,* the family *Deferribacteraceae,* the family *Fusobacteriaceae,* the family *Lactobacillaceae,* the family *Rhodocyclaceae,* the family *Verrucomicrobiaceae,* the family *Oxalobacteraceae,* the family *Nocardioidaceae,* the family *Propionibacteriaceae,* the family *Pasteurellaceae*, the family *Erysipelotrichaceae*, the family *Corynebacteriaceae,* the family *Prevotellaceae*, the family *Comamonadaceae*, the family *Rikenellaceae*, the family *Odoribacteraceae*, the family *Micrococcaceae*, the family *Microbacteriaceae*, the family *Alcaligenaceae*, the family *Methanobacteriaceae*, the family *Flavobacteriaceae*, and the family *Cryomorphaceae* as a biomarker exhibited significant diagnostic performance for stroke (see Table 5 and FIG. 5).

**[Table 5]**

| | Control | | Stroke | | t-test | | Training Set | | | Test Set | | |
|---|---|---|---|---|---|---|---|---|---|---|---|---|
| Taxon | Mean | SD | Mean | SD | p-value | Ratio | AUC | sensitivity | specificity | AUC | sensitivity | specificity |
| f_Dermacoccaceae | 0.0009 | 0.0025 | 0.0000 | 0.0001 | 0.0002 | 0.01 | 0.69 | 0.40 | 0.85 | 0.53 | 0.44 | 0.81 |
| f_Deferribacteraceae | 0.0020 | 0.0041 | 0.0001 | 0.0007 | 0.0000 | 0.03 | 0.66 | 0.31 | 0.89 | 0.61 | 0.53 | 0.89 |
| f_Fusobacteriaceae | 0.0018 | 0.0064 | 0.0001 | 0.0008 | 0.0071 | 0.06 | 0.69 | 0.34 | 0.89 | 0.54 | 0.34 | 0.83 |
| f_Lactobacillaceae | 0.0369 | 0.0355 | 0.0047 | 0.0225 | 0.0000 | 0.13 | 0.90 | 0.69 | 0.94 | 0.94 | 0.66 | 1.00 |
| f_Rhodocycl aceae | 0.0015 | 0.0045 | 0.0002 | 0.0010 | 0.0056 | 0.15 | 0.61 | 0.48 | 0.67 | 0.43 | 0.38 | 0.47 |
| f_Verrucomicrobiaceae | 0.0311 | 0.0439 | 0.0053 | 0.0184 | 0.0000 | 0.17 | 0.87 | 0.71 | 0.89 | 0.80 | 0.59 | 0.78 |
| f_ Oxalobacteraceae | 0.0148 | 0.0348 | 0.0025 | 0.0062 | 0.0004 | 0.17 | 0.66 | 0.48 | 0.76 | 0.55 | 0.34 | 0.83 |
| f_Nocardioidaceae | 0.0010 | 0.0023 | 0.0002 | 0.0012 | 0.0013 | 0.20 | 0.67 | 0.42 | 0.81 | 0.59 | 0.50 | 0.75 |
| f_Propionibacteriaceae | 0.0264 | 0.0325 | 0.0060 | 0.0110 | 0.0000 | 0.23 | 0.76 | 0.49 | 0.86 | 0.75 | 0.63 | 0.86 |
| f_Pasteurellaceae | 0.0046 | 0.0070 | 0.0012 | 0.0037 | 0.0000 | 0.27 | 0.72 | 0.45 | 0.84 | 0.62 | 0.50 | 0.81 |
| f_Erysipelotrichaceae | 0.0056 | 0.0104 | 0.0018 | 0.0054 | 0.0010 | 0.33 | 0.72 | 0.53 | 0.80 | 0.46 | 0.31 | 0.67 |
| f_ Corynebacteriaceae | 0.0504 | 0.0848 | 0.0225 | 0.0305 | 0.0016 | 0.45 | 0.65 | 0.48 | 0.71 | 0.53 | 0.38 | 0.69 |
| f_Prevotellaceae | 0.0174 | 0.0471 | 0.0407 | 0.0561 | 0.0010 | 2.34 | 0.63 | 0.61 | 0.57 | 0.51 | 0.47 | 0.47 |
| f_ Comamonadaceae | 0.0082 | 0.0136 | 0.0234 | 0.0344 | 0.0000 | 2.86 | 0.66 | 0.74 | 0.49 | 0.49 | 0.63 | 0.39 |
| f_Rikenellaceae | 0.0018 | 0.0048 | 0.0060 | 0.0119 | 0.0007 | 3.35 | 0.61 | 0.65 | 0.54 | 0.42 | 0.41 | 0.36 |
| f_ Odoribacteraceae | 0.0007 | 0.0022 | 0.0023 | 0.0058 | 0.0060 | 3.45 | 0.61 | 0.55 | 0.57 | 0.39 | 0.41 | 0.28 |
| f_Micrococcaceae | 0.0133 | 0.0156 | 0.0484 | 0.0539 | 0.0000 | 3.62 | 0.72 | 0.81 | 0.56 | 0.67 | 0.78 | 0.61 |
| f_Microbacteriaceae | 0.0022 | 0.0155 | 0.0099 | 0.0153 | 0.0002 | 4.58 | 0.71 | 0.74 | 0.56 | 0.57 | 0.59 | 0.44 |
| f_Alcaligenaceae | 0.0006 | 0.0020 | 0.0037 | 0.0105 | 0.0022 | 6.62 | 0.60 | 0.58 | 0.52 | 0.44 | 0.53 | 0.33 |
| f_Methanobacteriaceae | 0.0004 | 0.0017 | 0.0032 | 0.0077 | 0.0002 | 7.88 | 0.64 | 0.66 | 0.52 | 0.43 | 0.56 | 0.33 |
| f_Flavobacteriaceae | 0.0011 | 0.0024 | 0.0628 | 0.0883 | 0.0000 | 58.92 | 0.80 | 0.94 | 0.54 | 0.67 | 0.84 | 0.53 |
| f_ Cryomorphaceae | 0.0000 | 0.0000 | 0.0029 | 0.0068 | 0.0000 | >100 | 0.73 | 0.92 | 0.41 | 0.56 | 0.81 | 0.39 |

As a result of analyzing bacteria-derived EVs in blood at a genus level, a diagnostic model developed using bacteria belonging to the genus *Ralstonia*, the genus *Geobacillus*, the genus *Psychrobacter*, the genus *Cupriavidus*, the genus *Chromohalobacter*, the genus *Dermacoccus*, the genus *Enterobacter*, the genus *Jeotgalicoccus*, the genus *Mucispirillum,* the genus rc4-4, the genus *Fusobacterium*, the genus *Citrobacter*, the genus *Kocuria*, the genus *Lactobacillus*, the genus *Akkermansia*, the genus *Adlercreutzia*, the genus *Veillonella*, the genus *Propionibacterium*, the genus *Haemophilus*, the genus *Catenibacterium*, the genus *Klebsiella,* the genus *Corynebacterium,* the genus *Acinetobacter*, the genus *Prevotella*, the genus *Collinsella*, the genus *Micrococcus*, the genus *Methanobrevibacter*, the genus *Sutterella*, the genus *Lachnobacterium*, the genus *Limnohabitans*, the genus *Polynucleobacter*, the genus *Rhodobacter*, the genus *Flavobacterium,* the genus *Fluviicola*, and the genus *Candidatus Aquiluna* as a biomarker exhibited significant diagnostic performance for stroke (see Table 6 and FIG. 6).

**[Table 6]**

| | Control | | Stroke | | t-test | | Training Set | | | Test Set | | |
|---|---|---|---|---|---|---|---|---|---|---|---|---|
| Taxon | Mean | SD | Mean | SD | p-value | Ratio | AUC | sensitivity | specificity | AUC | sensitivity | specificity |
| g_Ralstonia | 0.0014 | 0.0049 | 0.0000 | 0.0000 | 0.0022 | 0.00 | 0.67 | 0.42 | 0.85 | 0.49 | 0.31 | 0.75 |
| g_Geobacillus | 0.0014 | 0.0044 | 0.0000 | 0.0000 | 0.0015 | 0.00 | 0.69 | 0.32 | 0.92 | 0.42 | 0.19 | 0.92 |
| g_ Psychrobacter | 0.0005 | 0.0017 | 0.0000 | 0.0000 | 0.0025 | 0.00 | 0.63 | 0.26 | 0.89 | 0.52 | 0.34 | 0.86 |
| g_Cupriavidus | 0.0099 | 0.0310 | 0.0001 | 0.0006 | 0.0013 | 0.01 | 0.74 | 0.48 | 0.90 | 0.60 | 0.31 | 0.92 |
| g_Chromohalobacter | 0.0024 | 0.0059 | 0.0000 | 0.0002 | 0.0001 | 0.01 | 0.64 | 0.25 | 0.94 | 0.56 | 0.41 | 0.92 |
| g_ Dermacoccus | 0.0009 | 0.0025 | 0.0000 | 0.0001 | 0.0002 | 0.01 | 0.69 | 0.40 | 0.85 | 0.53 | 0.44 | 0.81 |
| g_ Enterobacter | 0.0037 | 0.0140 | 0.0000 | 0.0002 | 0.0073 | 0.01 | 0.72 | 0.40 | 0.94 | 0.57 | 0.44 | 0.94 |
| g_ Jeotgalicoccus | 0.0006 | 0.0017 | 0.0000 | 0.0001 | 0.0004 | 0.01 | 0.70 | 0.36 | 0.91 | 0.41 | 0.09 | 0.94 |
| g_ Mucispirillum | 0.0020 | 0.0041 | 0.0001 | 0.0007 | 0.0000 | 0.03 | 0.66 | 0.31 | 0.89 | 0.61 | 0.53 | 0.89 |
| g_rc4-4 | 0.0017 | 0.0055 | 0.0001 | 0.0008 | 0.0024 | 0.04 | 0.67 | 0.25 | 0.99 | 0.57 | 0.31 | 1.00 |
| g_ Fusobacterium | 0.0018 | 0.0064 | 0.0001 | 0.0008 | 0.0072 | 0.06 | 0.69 | 0.34 | 0.89 | 0.54 | 0.34 | 0.83 |
| g_Citrobacter | 0.0078 | 0.0112 | 0.0005 | 0.0025 | 0.0000 | 0.06 | 0.82 | 0.55 | 0.90 | 0.71 | 0.44 | 1.00 |
| g_Kocuria | 0.0007 | 0.0017 | 0.0001 | 0.0007 | 0.0005 | 0.11 | 0.67 | 0.30 | 0.92 | 0.59 | 0.31 | 0.94 |
| g_ Lactobacillus | 0.0361 | 0.0354 | 0.0046 | 0.0225 | 0.0000 | 0.13 | 0.89 | 0.68 | 0.94 | 0.94 | 0.69 | 1.00 |
| g_ Akkermansia | 0.0311 | 0.0439 | 0.0050 | 0.0184 | 0.0000 | 0.16 | 0.88 | 0.71 | 0.90 | 0.81 | 0.63 | 0.78 |
| g_ Adlercreutzia | 0.0019 | 0.0039 | 0.0003 | 0.0020 | 0.0002 | 0.16 | 0.74 | 0.38 | 0.95 | 0.51 | 0.25 | 0.94 |
| g_Veillonella | 0.0062 | 0.0181 | 0.0011 | 0.0035 | 0.0047 | 0.17 | 0.70 | 0.45 | 0.82 | 0.49 | 0.31 | 0.83 |
| g_ Propionibacterium | 0.0264 | 0.0325 | 0.0060 | 0.0110 | 0.0000 | 0.23 | 0.76 | 0.49 | 0.86 | 0.75 | 0.63 | 0.86 |
| g_ Haemophilus | 0.0041 | 0.0067 | 0.0010 | 0.0032 | 0.0000 | 0.25 | 0.72 | 0.43 | 0.84 | 0.61 | 0.50 | 0.81 |
| g_Catenibacterium | 0.0030 | 0.0071 | 0.0008 | 0.0042 | 0.0054 | 0.26 | 0.67 | 0.60 | 0.70 | 0.42 | 0.31 | 0.47 |
| g_ Klebsiella | 0.0068 | 0.0125 | 0.0019 | 0.0059 | 0.0003 | 0.27 | 0.72 | 0.52 | 0.85 | 0.53 | 0.31 | 0.83 |
| g_Corynebacterium | 0.0504 | 0.0848 | 0.0225 | 0.0305 | 0.0016 | 0.45 | 0.65 | 0.48 | 0.71 | 0.53 | 0.38 | 0.69 |
| g_ Acinetobacter | 0.0594 | 0.0803 | 0.0280 | 0.0573 | 0.0010 | 0.47 | 0.66 | 0.47 | 0.68 | 0.49 | 0.41 | 0.67 |
| g_ Prevotella | 0.0174 | 0.0471 | 0.0407 | 0.0561 | 0.0010 | 2.34 | 0.63 | 0.61 | 0.57 | 0.51 | 0.47 | 0.47 |
| g_Collinsella | 0.0027 | 0.0061 | 0.0083 | 0.0221 | 0.0091 | 3.13 | 0.62 | 0.58 | 0.54 | 0.41 | 0.50 | 0.33 |
| g_ Micrococcus | 0.0061 | 0.0092 | 0.0418 | 0.0513 | 0.0000 | 6.89 | 0.75 | 0.88 | 0.54 | 0.67 | 0.75 | 0.67 |
| g_ Methanobrevibacter | 0.0004 | 0.0017 | 0.0032 | 0.0077 | 0.0002 | 8.10 | 0.64 | 0.66 | 0.52 | 0.44 | 0.53 | 0.33 |
| g_Sutterella | 0.0004 | 0.0016 | 0.0031 | 0.0086 | 0.0011 | 8.71 | 0.62 | 0.62 | 0.51 | 0.44 | 0.50 | 0.33 |
| g_ Lachnobacterium | 0.0001 | 0.0003 | 0.0018 | 0.0067 | 0.0066 | 25.14 | 0.62 | 0.66 | 0.49 | 0.35 | 0.53 | 0.22 |
| g_ Limnohabitans | 0.0000 | 0.0002 | 0.0006 | 0.0025 | 0.0087 | 33.61 | 0.63 | 0.68 | 0.47 | 0.41 | 0.56 | 0.28 |
| g_ Polynucleobacter | 0.0000 | 0.0001 | 0.0015 | 0.0041 | 0.0002 | 100.77 | 0.71 | 0.75 | 0.54 | 0.36 | 0.63 | 0.19 |
| g_ Rhodobacter | 0.0000 | 0.0002 | 0.0033 | 0.0065 | 0.0000 | 140.13 | 0.76 | 0.99 | 0.44 | 0.57 | 0.88 | 0.42 |
| g_ Flavobacterium | 0.0004 | 0.0015 | 0.0626 | 0.0883 | 0.0000 | 140.16 | 0.82 | 0.94 | 0.54 | 0.69 | 0.81 | 0.53 |
| g_Fluviicola | 0.0000 | 0.0000 | 0.0028 | 0.0066 | 0.0000 | >100 | 0.73 | 0.92 | 0.41 | 0.56 | 0.81 | 0.39 |
| g_Candidatus Aquiluna | 0.0000 | 0.0000 | 0.0005 | 0.0019 | 0.0059 | >100 | 0.63 | 0.74 | 0.46 | 0.43 | 0.56 | 0.28 |

The above description of the present invention is provided only for illustrative purposes, and it will be understood by one of ordinary skill in the art to which the present invention pertains that the invention may be embodied in various modified forms without departing from the spirit or essential characteristics thereof. Thus, the embodiments described herein should be considered in an illustrative sense only and not for the purpose of limitation.

### [Industrial Applicability]

The method of providing information for diagnosing stroke through a bacterial metagenomic analysis according to the present invention may be used for predicting the risk of stroke onset and diagnosing stroke by performing a bacterial metagenomic analysis using subject-derived samples to analyze an increase or decrease in the content of specific bacteria-derived extracellular vesicles.

## Claims

1. A method of providing information for stroke diagnosis, comprising the following processes:
(a) extracting DNAs from extracellular vesicles isolated from a subject sample;
(b) performing PCR on the extracted DNA using a pair of primers comprising SEQ ID NO: 1 and SEQ ID NO: 2; and
(c) comparing an increase or decrease in contents of bacteria-derived extracellular vesicles of the subject-derived sample with that of a normal individual-derived sample through sequencing of a product of the PCR.

2. The method of claim 1, wherein process (c) comprises comparing an increase or decrease in content of extracellular vesicles derived from one or more bacteria selected from the group consisting of the phylum *Deferribacteres*, the phylum *Verrucomicrobia*, the phylum *Tenericutes*, the phylum *Cyanobacteria*, the phylum *Bacteroidetes*, the phylum *Euryarchaeota*, the phylum TM7, the phylum *Planctomycetes*, the phylum GN02, and the phylum OD1.

3. The method of claim 1, wherein process (c) comprises comparing an increase or decrease in content of extracellular vesicles derived from one or more bacteria selected from the group consisting of the class *Halobacteria,* the class *Deferribacteres,* the class *Verrucomicrobiae*, the class *Chloroplast*, the class *Mollicutes*, the class *Erysipelotrichi,* the class *Methanobacteria*, the class *Flavobacteriia*, the class *Planctomycetia*, the class TM7-1, and the class ZB2.

4. The method of claim 1, wherein process (c) comprises comparing an increase or decrease in content of extracellular vesicles derived from one or more bacteria selected from the group consisting of the order *Halobacteriales,* the order *Deferribacterales,* the order *Streptophyta*, the order *Rhodocyclales*, the order *Verrucomicrobiales*, the order *Rickettsiales*, the order *Pasteurellales,* the order *Erysipelotrichales,* the order *Methanobacteriales*, the order *Legionellales,* and the order *Flavobacteriales.*

5. The method of claim 1, wherein process (c) comprises comparing an increase or decrease in content of extracellular vesicles derived from one or more bacteria selected from the group consisting of the family *Dermacoccaceae*, the family *Deferribacteraceae*, the family *Fusobacteriaceae*, the family *Lactobacillaceae*, the family *Rhodocyclaceae*, the family *Verrucomicrobiaceae*, the family *Oxalobacteraceae*, the family *Nocardioidaceae*, the family *Propionibacteriaceae*, the family *Pasteurellaceae*, the family *Erysipelotrichaceae*, the family *Corynebacteriaceae*, the family *Prevotellaceae*, the family *Comamonadaceae*, the family *Rikenellaceae*, the family *Odoribacteraceae*, the family *Micrococcaceae*, the family *Microbacteriaceae*, the family *Alcaligenaceae*, the family *Methanobacteriaceae*, the family *Flavobacteriaceae*, and the family *Cryomorphaceae.*

6. The method of claim 1, wherein process (c) comprises comparing an increase or decrease in content of extracellular vesicles derived from one or more bacteria selected from the group consisting of the genus *Ralstonia*, the genus *Geobacillus*, the genus *Psychrobacter*, the genus *Cupriavidus*, the genus *Chromohalobacter*, the genus *Dermacoccus*, the genus *Enterobacter*, the genus *Jeotgalicoccus*, the genus *Mucispirillum*, the genus rc4-4, the genus *Fusobacterium*, the genus *Citrobacter*, the genus *Kocuria*, the genus *Lactobacillus*, the genus *Akkermansia*, the genus *Adlercreutzia*, the genus *Veillonella*, the genus *Propionibacterium*, the genus *Haemophilus,* the genus *Catenibacterium*, the genus *Klebsiella,* the genus *Corynebacterium,* the genus *Acinetobacter*, the genus *Prevotella,* the genus *Collinsella*, the genus *Micrococcus*, the genus *Methanobrevibacter*, the genus *Sutterella*, the genus *Lachnobacterium*, the genus *Limnohabitans*, the genus *Polynucleobacter*, the genus *Rhodobacter*, the genus *Flavobacterium,* the genus *Fluviicola*, and the genus *Candidatus Aquiluna.*

7. The method of claim 1, wherein the subject sample is blood.

8. The method of claim 7, wherein the blood is whole blood, serum, plasma, or blood mononuclear cells.

9. A method of diagnosing stroke, comprising the following processes:
(a) extracting DNAs from extracellular vesicles isolated from a subject sample;
(b) performing PCR on the extracted DNA using a pair of primers comprising SEQ ID NO: 1 and SEQ ID NO: 2; and
(c) comparing an increase or decrease in contents of bacteria-derived extracellular vesicles of the subject-derived sample with that of a normal individual-derived sample through sequencing of a product of the PCR.

10. The method of claim 9, wherein process (c) comprises comparing an increase or decrease in content of extracellular vesicles derived from one or more bacteria selected from the group consisting of the phylum *Deferribacteres*, the phylum *Verrucomicrobia*, the phylum *Tenericutes*, the phylum *Cyanobacteria,* the phylum *Bacteroidetes*, the phylum *Euryarchaeota*, the phylum TM7, the phylum *Planctomycetes*, the phylum GN02, and the phylum OD1.

11. The method of claim 9, wherein process (c) comprises comparing an increase or decrease in content of extracellular vesicles derived from one or more bacteria selected from the group consisting of the class *Halobacteria*, the class *Deferribacteres,* the class *Verrucomicrobiae*, the class *Chloroplast,* the class *Mollicutes*, the class *Erysipelotrichi,* the class *Methanobacteria*, the class *Flavobacteriia*, the class *Planctomycetia*, the class TM7-1, and the class ZB2.

12. The method of claim 9, wherein process (c) comprises comparing an increase or decrease in content of extracellular vesicles derived from one or more bacteria selected from the group consisting of the order *Halobacteriales,* the order *Deferribacterales,* the order *Streptophyta*, the order *Rhodocyclales*, the order *Verrucomicrobiales*, the order *Rickettsiales*, the order *Pasteurellales*, the order *Erysipelotrichales*, the order *Methanobacteriales*, the order *Legionellales*, and the order *Flavobacteriales.*

13. The method of claim 9, wherein process (c) comprises comparing an increase or decrease in content of extracellular vesicles derived from one or more bacteria selected from the group consisting of the family *Dermacoccaceae*, the family *Deferribacteraceae*, the family *Fusobacteriaceae*, the family *Lactobacillaceae*, the family *Rhodocyclaceae*, the family *Verrucomicrobiaceae*, the family *Oxalobacteraceae*, the family *Nocardioidaceae*, the family *Propionibacteriaceae*, the family *Pasteurellaceae*, the family *Erysipelotrichaceae*, the family *Corynebacteriaceae*, the family *Prevotellaceae*, the family *Comamonadaceae*, the family *Rikenellaceae,* the family *Odoribacteraceae*, the family *Micrococcaceae*, the family *Microbacteriaceae*, the family *Alcaligenaceae*, the family *Methanobacteriaceae*, the family *Flavobacteriaceae*, and the family *Cryomorphaceae.*

14. The method of claim 9, wherein process (c) comprises comparing an increase or decrease in content of extracellular vesicles derived from one or more bacteria selected from the group consisting of the genus *Ralstonia*, the genus *Geobacillus*, the genus *Psychrobacter*, the genus *Cupriavidus*, the genus *Chromohalobacter*, the genus *Dermacoccus*, the genus *Enterobacter*, the genus *Jeotgalicoccus*, the genus *Mucispirillum*, the genus rc4-4, the genus *Fusobacterium*, the genus *Citrobacter*, the genus *Kocuria,* the genus *Lactobacillus,* the genus *Akkermansia*, the genus *Adlercreutzia*, the genus *Veillonella*, the genus *Propionibacterium*, the genus *Haemophilus*, the genus *Catenibacterium,* the genus *Klebsiella,* the genus *Corynebacterium*, the genus *Acinetobacter*, the genus *Prevotella*, the genus *Collinsella*, the genus *Micrococcus,* the genus *Methanobrevibacter*, the genus *Sutterella*, the genus *Lachnobacterium*, the genus *Limnohabitans*, the genus *Polynucleobacter*, the genus *Rhodobacter*, the genus *Flavobacterium*, the genus *Fluviicola*, and the genus *Candidatus Aquiluna.*

15. The method of claim 9, wherein the subject sample is blood.

16. The method of claim 15, wherein the blood is whole blood, serum, plasma, or blood mononuclear cells.
